# FASCICULE DE BREVET EUROPEEN

(11) **EP 2 012 792 B1**
(45) Date de publication et mention de la délivrance du brevet: **08.04.2015**
(21) Numéro de dépôt: 07731936.6
(22) Date de dépôt: 24.04.2007
(51) Int. Cl.: A61K 31/55, A61K 31/138, A61P 21/00

(54) **UTILISATION D'UN COMPOSE QUI INDUIT UNE AUGMENTATION DU TAUX DE SEROTONINE LIBRE DANS L'ORGANISME, POUR LA PREPARATION D'UN MEDICAMENT DESTINE A TRAITER DES DEGENERESCENCES DU MUSCLE SQUELETTIQUE D'ORIGINE GENETIQUE**
VERWENDUNG EINER VERBINDUNG ZUR AUSLÖSUNG EINES ANSTIEGS DER FREIEN SEROTONIN-RATE IM KÖRPER, ZUR HERSTELLUNG EINES MEDIKAMENTS ZUR BEHANDLUNG VON SKELETTMUSKELDEGENERATION AUS GENETISCHEM GRUND
USE OF A COMPOUND INDUCING AN INCREASE OF FREE SEROTONIN RATE IN THE BODY, FOR PREPARING A MEDICINE FOR TREATING SKELETAL MUSCLE DEGENERATION OF GENETIC ORIGIN

(30) Priorité: 28.04.2006 FR 0651516
(43) Date de publication de la demande: 14.01.2009
(73) Titulaire: Association Française contre les Myopathies, 91002 Evry (FR)
(72) Inventeur: SEGALAT, Laurent, F-69340 Francheville (FR)
(74) Mandataire: Sarlin, Laure V.
(86) Numéro de dépôt international: PCT/FR2007/051163
(87) Numéro de publication internationale: WO 2007/125245

(56) Documents cités:
- CA-A- 2 292 874
- WANG ET AL.: "Lack of effects by tricyclic antidepressant and serotoinin inhibitors on anorexia in MCG 101 tumor-bearing mice with eicosanoid-related cachexia" NUTRITION, vol. 19, 2003, pages 47-53, XP002412735
- MOXLEY ET AL.: "Practice parameter: corticosteroid treatment of Duchenne dystrophy" NEUROLOGY, vol. 64, 2005, pages 13-20, XP002412736

## Description

La présente invention a pour objet l'utilisation de composés antidépresseurs qui permettent d'augmenter le taux de sérotonine libre dans l'organisme, pour la préparation d'un médicament destiné à traiter des pathologies correspondant à une dégénérescence du muscle squelettique d'origine génétique.

La publication Nutrition, 2003, 19 : 47-53 rapporte l'absence d'effet d'antidépresseurs tricycliques et d'inhibiteurs de la sérotonine sur l'anorexie, chez des souris MCG 101 porteuses de tumeurs avec une cachexie liée aux eicosanoides.

Les myopathies dégénératives (ou atrophies musculaires) d'origine génétique sont des maladies héréditaires dues à des mutations dans des gènes codant pour des protéines musculaires. Il existe plusieurs dizaines de myopathies dégénératives d'origine génétique. Bien que le tableau clinique puisse varier entre deux maladies, elles ont en commun de conduire inexorablement à l'atrophie musculaire des muscles dits squelettiques. Cette atrophie musculaire (aussi appelée fonte musculaire) se traduit par un handicap de plus en plus important pour le patient, qui peut conduire à la mort lorsque les muscles respiratoires sont atteints. La plus connue d'entre les myopathies dégénératives d'origine génétique est la myopathie de Duchenne (DMD). Il n'existe pas à ce jour de traitement efficace contre ces maladies.

Les cachexies, myopathies dégénératives (ou atrophies musculaires), non génétiques correspondent aux maladies conduisant à une fonte musculaire, indépendamment de l'atteinte primaire. La cachexie est notamment observée dans la plupart des cancers. Il n'existe pas de traitement efficace contre la cachexie cancéreuse.

Il existe donc un besoin pour de nouveaux moyens de traiter ces maladies et pour de nouveaux médicaments adaptés à de tels traitements.

Dans ce contexte et de façon totalement inattendue, l'inventeur de la présente demande de brevet a mis en évidence que les composés permettant d'augmenter le taux de sérotonine libre dans l'organisme, jusqu'alors connus en général pour leur activité d'antidépresseur, permettaient de réduire la fonte des muscles squelettiques.

Par conséquent, la présente invention a pour objet l'utilisation d'un composé qui induit une augmentation du taux de sérotonine libre dans l'organisme, pour la préparation d'un médicament destiné à traiter ou prévenir des pathologies correspondant à une dégénérescence musculaire des muscles squelettiques d'origine génétique, comme les myopathies musculaires.

En particulier, l'utilisation des composés capables d'augmenter le taux de sérotonine dans l'organisme est particulièrement adaptée à la préparation de médicaments destinés à traiter les myopathies dégénératives d'origine génétique, et notamment la myopathie de Duchenne.

La sérotonine ou 5-hydrotryptamine (5-HT) est un messager chimique du système nerveux central intervenant dans de nombreuses fonctions physiologiques. Notamment, la sérotonine apparaît être un neuromédiateur essentiel dans la dépression.

Les récepteurs de la sérotonine sont classés en 7 groupes 5-HT1, 5-HT2, 5-HT3, 5-HT4, 5-HT5, 5-HT6 et 5-HT7, chaque groupe pouvant avoir des sous-classes A, B ... Ces récepteurs sont localisés au niveau du cerveau et à la périphérie, mais leur distribution n'est pas homogène.

Comme les autres médiateurs, la sérotonine libérée dans la fente synaptique est en grande partie recaptée par les terminaisons présynaptiques, grâce à un transporteur spécifique qui présente un assez grand polymorphisme génétique.

Il existe trois grandes classes de composés qui peuvent être utilisés dans le cadre de l'invention et qui permettent d'augmenter le taux de sérotonine, dans l'organisme d'un être vivant, et en particulier chez l'homme. Ce taux est, en particulier, augmenté dans la synapse.

On peut distinguer :
- les inhibiteurs sélectifs de la recapture de la sérotonine qui augmentent la concentration de sérotonine dans la synapse en empêchant sa recapture dans le neurone récepteur. A titre d'exemple de tels composés, on peut citer :
   - le citalopram, commercialisée notamment sous le nom de *Seropram*^{®} ;
   - la fluoxétine de formule : 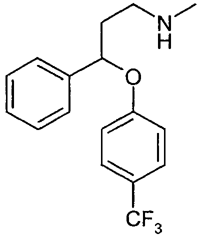 en particulier sous forme chrorhydrate, commercialisée notamment sous le nom de Prozac^{®} ;
   - la norfluoxétine, métabolite de la fluoxétine ;
   - la fluvoxamine, en particulier sous forme maléate, commercialisée notamment sous le nom de Floxyfral^{®} ;
   - l'escitalopram, en particulier sous forme oxalate, commercialisé notamment sous le nom de Seroplex^{®} ;
   - la paroxétine, commercialisée notamment sous le nom de Deroxat^{®}, Divarius^{®} ;
   - la sertraline commercialisée notamment sous le nom de Zoloft^{®} ;
   - l'alaproclate, l'étopéridone, l'escitalopram et la zimélidine ;
- les composés tricycliques ou tétracycliques, par exemple de la famille des phénothiazines ou des dibenzazépines, qui empêchent la recapture de divers neurotransmetteurs, dont la sérotonine, la noradrénaline et la dopamine et ne sont donc pas, contrairement aux précédents, sélectifs de la sérotonine. Ces composés sont, en général, connus pour leur effet antidépresseur. A titre d'exemple de tels composés, on peut citer, comme composés tricycliques, l'amitriptyline (en particulier sous la forme chlorhydrate) de formule : 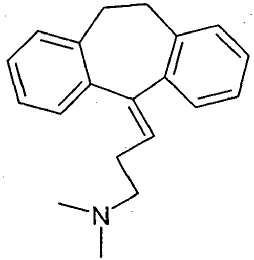 la clomipramine, la désipramine (en particulier sous la forme chlorhydrate) de formule : 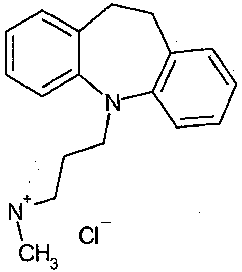 la dothiépine, la doxépine, l'imipramine (en particulier sous la forme chlorhydrate) de formule : 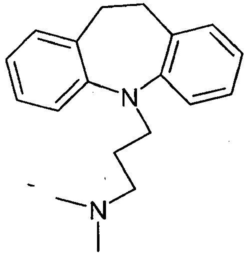 la lofépramine, la nortriptyline, la protriptyline, la trimipramine (en particulier sous la forme maléate) de formule : 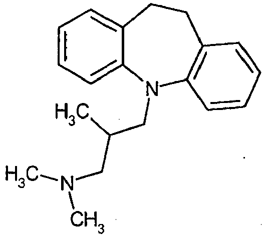 l'iprindole et l'opipramol et comme composés tétracycliques, la maprotiline, la miansérine, la mirtazapine et l'amoxapine ; Certains de ces dérivés tricycliques sont porteurs d'un groupe amine tertiaire diméthylée ou amine secondaire monométhylée ;
- les inhibiteurs des monoamine oxydases qui augmentent la concentration en sérotonine en inhibant les monoamine oxydases, enzymes chargées de la dégradation de la sérotonine. A titre d'exemple de tels composés, on peut citer la moclobémide, la phénelzine, l'harmaline, la nialamide, la sélégiline, l'isocarboxazide, llproniazide, l'iproclozide, la toloxatone et la tranylcypromine.

A titre d'exemples de tests permettant de vérifier si un composé est capable d'augmenter le taux de sérotonine dans l'organisme d'un être vivant, on peut citer des tests de réabsorption de la sérotonine dans le cerveau de rat *in vitro* décrits dans les publications suivantes :
- Shank RP, Vaught JL, Pelley KA, Setler PE, McComsey DF, Maryanoff BE, McN-5652: a highly potent inhibitor of serotonin uptake. J Pharm Exp Therap. 1988, 247, 1032-1038
- Hyttel J, Comparative pharmacology of selective serotonin reuptake inhibitors (SSRIs). Nord J Psychiatry. 1993, 47 (suppl 30), 5-12.

Selon une variante de l'invention, les composés utilisés dans le cadre de l'invention présenteront sur au moins l'un de ces tests, ou un test similaire, une IC50 inférieure à 10µM, de préférence inférieure ou égale à 1 µM et préférentiellement inférieure ou égale à 100 nM.

Les composés listés précédemment sont en général des médicaments commercialisés et pourront être administrés selon des formes pharmaceutiques connues pour d'autres pathologies. Les composés sont conditionnés en mélange avec un ou plusieurs excipients pharmaceutiquement acceptables et les composés peuvent se trouver sous la forme de sels, solvats ou hydrates pharmaceutiquement acceptables. Ces compositions pharmaceutiques sont préparées selon les techniques classiques bien connues de l'homme de l'art. Les excipients pharmaceutiques sont choisis selon la forme pharmaceutique et le mode d'administration souhaité, par exemple, administration orale, sublinguale, sous-cutanée, intramusculaire, intra-veineuse, topique ... A titre d'exemple d'excipients pharmaceutiquement acceptables, on peut citer la silice colloïdale anhydre, glycérol, lactose, magnésium stéarate, amidon de maïs, acide stéarique, talc, hypromellose, polymère de vinylpyrrolidone et vinylacétate, cellulose microcristalline, macrogol 8000, povidone K 30, saccharose, dispersion de fer rouge oxyde dans le dioxyde de titane, titane dioxyde, saccharine sodique, mannitol, sorbitol, anis arôme, menthe arôme, sodium stéarylfumarate...

Les compositions pharmaceutiques pour l'administration orale, sublinguale, sous-cutanée, intramusculaire, intra-veineuse, topique, intratrachéale, intranasale, transdermique, rectale ou intraoculaire, comprenant le composé actif, peuvent être administrées sous formes unitaires d'administration, en mélange avec des supports pharmaceutiques classiques, aux animaux et aux êtres humains pour la prophylaxie ou le traitement des dégénérescences des muscles squelettiques. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades, lotions ou collyres.

La dose sera adaptée à l'effet prophylactique ou thérapeutique souhaité, à l'age et au poids du patient à traiter. De préférence, la dose journalière sera comprise entre 10 et 500 mg/kg/jour et répartie en 1 à 3 prises. Afin d'obtenir l'effet prophylactique ou thérapeutique désiré, chaque dose unitaire peut contenir de 1 à 500 mg, de composé actif en combinaison avec un support pharmaceutique. Cette dose unitaire peut être administrée 1 à 5 fois par jour de façon à administrer un dosage journalier permettant d'obtenir l'effet souhaité.

Lorsqu'on prépare une composition solide sous forme de comprimés, on mélange l'ingrédient actif principal avec un véhicule pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié. Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs de goût.

Pour une administration rectale, on recourt à des suppositoires qui sont préparés avec des liants fondant à la température rectale, par exemple du beurre de cacao ou des polyéthylèneglycols. Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol. Le principe actif peut être formulé également sous forme de microcapsules, éventuellement ave un ou plusieurs supports ou additifs, ou bien avec des matrices telles qu'un polymère ou une cyclodextrine (patch, formes à libération prolongée).

Certains des composés permettant d'augmenter le taux de sérotonine, précédemment cités, font déjà l'objet de médicaments commercialisés et pourront donc être utilisés sous des formulation pharmaceutiques déjà connues pour d'autres indications.

La fluoxétine sous forme chlorhydrate est commercialisée, notamment, sous le nom de Prozac®.

L'imipramine chlorhydrate est, par exemple, commercialisée sous les noms de Antideprin®, Janimine®, Tofranil®.

L'amitriptyline chlorhydrate est, par exemple, commercialisée sous les noms de Elavil®, Tryptanol®, Endep®, Tryptizol®.

La désipramine chlorhydrate est, par exemple, commercialisée sous les noms de Pertrofane® et Nopramine®.

La trimipramine maléate est, par exemple, commercialisée sous les noms de Surmontril® et Rhotrimine®.

Pour plus de détails sur ces compositions, on pourra consulter le VIDAL dans son Edition 2006.

L'activité de ces composés et leur action sur la fonte des muscles squelettiques ont été mises en évidence, par l'inventeur, dans un test effectué chez les nématodes qui jouent le rôle de modèle pour les myopathies dégénératives (Gaud A. et al. Prednisone reduces muscle degeneration in dystrophin-deficient Caenorhabditis elegans, Neuromuscul. Disord. 2004, 14, 365-70). Pour certains composés, l'activité constatée chez les nématodes a été confirmée chez la souris.

Les résultats ci-après sont donnés à titre d'illustration.

### Tests sur le modèle animal Caenorhabditis elegans

### a) Méthode

Les composés ont été testés sur une souche de nématode dépourvue de dystrophine (Gieseler et al., Current Biology, 2000, vol. 10, p1092-1097) et (Ségalat, Neuromuscular Disorders, 2002, Suppl: S105-9). Cette souche est utilisée comme modèle de myopathie dégénérative. Dans cette souche, jusqu'à 30% des cellules musculaires servant à la locomotion (muscles longitudinaux) dégénèrent à l'état adulte (7 jours à 15°C), ce qui se traduit par une paralysie progressive des animaux.

Les composés sont administrés par mélange avec la gélose sur laquelle sont cultivés les animaux. Ils pénètrent par diffusion à travers la cuticule des animaux.

L'effet des composés sur la dégénérescence musculaire est évalué par 1) l'observation du phénotype des animaux (paralysie), et 2) par l'examen cytologique de la dégénérescence musculaire au moyen de marqueurs musculaires : rodamine-phalloidine et anticorps anti-myosine. Cet examen permet de compter le nombre de cellules musculaires en dégénérescence ou mortes (Gieseler *et al*., 2000, *supra*).

### b) Résultats

Le tableau suivant donne les résultats obtenus en ce qui concerne la locomotion et la dégénérescence des animaux après 7 jours de culture à 15°C. Chacun des composés diminue la proportion d'animaux paralysés et la dégénérescence musculaire comme le montre le tableau ci-dessous. Par ailleurs, la sérotonine a été testée et diminue également la dégénérescence musculaire. Ce résultat prouve bien que l'augmentation du taux de sérotonine libre dans l'organisme est directement liée à l'effet des composés.

| **Composé testé** | **Concentration (mg/ml)** | **Concentration en mM** | **Pourcentage d'animaux paralysés à J7** | **Diminution de la dégénérescence en %** |
|---|---|---|---|---|
| Amytriptyline chlorhydrate | 0,1 mg/ml | 0,3 mM | 52 | 61 |
| Desipramine chlorhydrate | 0,1 mg/ml | 0,3 mM | 58 | 58 |
| Trimipramine maleate | 0,1 mg/ml | 0,2 mM | 60 | 53 |
| Imipramine chlorhydrate | 0,1 mg/ml | 0,3 mM | 65 | 37 |
| Fluoxetine chlorhydrate | 0,05 mg/ml | 0,15 mM | 60 | 51 |
| Sérotonine | 4 mg/ml | 10 mM | 41 | 73 |
| Non traité | - | - | 100 | 0 |

Note : les concentrations figurant dans ce tableau sont les concentrations dans la gélose de culture. Les concentrations à l'intérieur de l'animal sont inconnues, car la méthode ne permet pas de les mesurer précisément. Les concentrations à l'intérieur de l'animal sont estimées à 100 à 1000 fois inférieures à celles du milieu de la gélose.

c) Concernant l'action sur la cachexie, il a également pu être montré sur un modèle de protéolyse du muscle de C. elegans representatif de la cachexie, et en utilisant le protocole décrit par Zdinak et al., dans Transgene-coded chimeric proteins as reporters of intracellular proteolysis: starvation-induced catabolism of a lacZ fusion protein in muscle cells of Caenorhabditis elegans, J Cell Biochem. 1997 Oct 1;67(1):143-53 que la sérotonine et l'imipramine chlorhydrate réduisent fortement cette protéolyse (20% de la protéolyse du témoin dans les 2 cas).

### Tests chez la souris

### Méthode :

Les tests effectués chez la souris sont de plusieurs types : histologiques, fonctionnels sur l'animal entier, et fonctionnels sur muscles disséqués. Les tests sont effectués sur la souris mdx (génotype C57/BL6 mdx5cv), modèle murin de myopathie dégénérative. Ces tests sont notamment détaillés dans les publications suivantes auxquelles on pourra se référer :
De Luca A, et al. Am. J. Pathol. 2005 Feb ; 166(2), 477-89.
Divet A, et al. Pflugers Arch. 2002 Aug; 444(5), 634-43. Epub 2002 Jul 10.
Rafael JA, et al. Mamm Genome. 2000 Sep ; 11(9), 725-8.

Les composés sont administrés oralement aux souris dans leur nourriture. Les souris sont traitées de l'âge de 2 semaines à 6 semaines. Elles sont testées à 6 semaines.

### Imipramine chlorhydrate (40mg/jour/kg suivant les expériences)

- Réduction de 26% de la dispersion de taille des fibres (marqueur de dégénérescence) par rapport aux animaux non traités.
- Augmentation de 48% du score avec le test d'agrippement.
- Augmentation de 20% de la tension développée par le muscle EDL sur animaux vivants
- Augmentation de 50% de la tension maximale développée par le muscle isolé (protocole de fibres pelées au triton).

Il a également été démontré que la Trimipramine maléate (15 mg/kg/jour) réduit la nécrose du diaphragme (selon le test décrit par Raymackers JM et al, dans Consequence of parvalbumin deficiency in the mdx mouse: histological, biochemical and mechanical phenotype of a new double mutant. Neuromuscul Disord. 2003 Jun;13(5):376-87), ainsi que le nombre de fibres centronucléées (respectivement, selon le test décrit par Shavlakadze T, et al. Targeted expression of insulin-like growth factor-I reduces early myofiber necrosis in dystrophic mdx mice. Mol Ther. 2004 Nov;10(5):829-43.), 2 indicateurs de la myopathie : p<5% et p<0,1% respectivement. Il a également été démontré que la Fluoxetine chlorhydrate (11 mg/kg/jour), réduit le nombre de fibres centronucléées de 20% environ (p<0,1%).

## Revendications

1. Composé qui induit une augmentation du taux de sérotonine libre dans l'organisme, pour son utilisation dans le traitement ou la prévention de pathologies correspondant à une dégénérescence musculaire des muscles squelettiques choisies parmi les myopathies musculaires d'origine génétique.

2. Utilisation d'un composé qui induit une augmentation du taux de sérotonine libre dans l'organisme, pour la préparation d'un médicament destiné au traitement ou à la prévention de pathologies correspondant à une dégénérescence musculaire des muscles squelettiques choisies parmi les myopathies musculaires d'origine génétique.

3. Composé pour utilisation selon la revendication 1 ou utilisation selon la revendication 2 caractérisé que le composé est un inhibiteur sélectif de la recapture de la sérotonine.

4. Composé pour utilisation ou utilisation selon la revendication 3 **caractérisé en ce que** le composé est choisi parmi : le citalopram, la fluoxétine de formule : 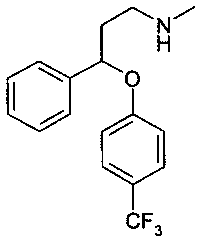 en particulier sous forme chrorhydrate, la norfluoxétine, la fluvoxamine en particulier sous forme maléate, l'escitalopram en particulier sous forme oxalate, la paroxétine, la sertraline, l'alaproclate, l'étopéridone, l'escitalopram et la zimélidine.

5. Composé pour utilisation selon la revendication 1 ou utilisation selon la revendication 2 **caractérisé en ce que** le composé est un composé tricyclique ou tétracyclique qui empêche la recapture de divers neurotransmetteurs, dont la sérotonine, la noradrénaline et la dopamine.

6. Composé pour utilisation ou utilisation selon la revendication 5 **caractérisé en ce que** le composé appartient à la famille des phénothiazines ou des dibenzazépines.

7. Composé pour utilisation ou utilisation selon la revendication 5 **caractérisé en ce que** le composé est choisi parmi l'amitriptyline, en particulier sous la forme chlorhydrate, de formule : 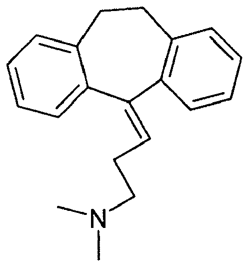 la clomipramine, la désipramine, en particulier sous la forme chlorhydrate, de formule : 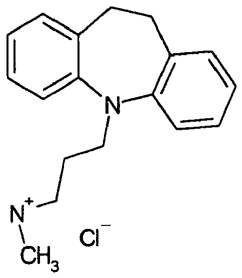 la dothiépine, la doxépine, l'imipramine, en particulier sous la forme chlorhydrate, de formule : 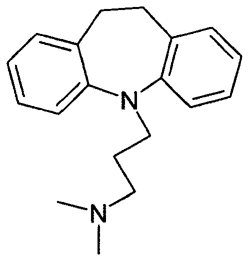 la lofépramine, la nortriptyline, la protriptyline, la trimipramine, en particulier sous la forme maléate, de formule : 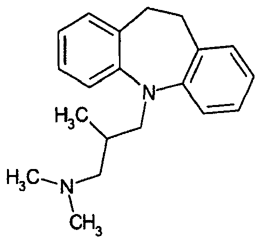 l'iprindole, l'opipramol, la maprotiline, la miansérine, la mirtazapine et l'amoxapine.

8. Composé pour utilisation selon la revendication 1 ou utilisation selon la revendication 2 caractérisé que le composé est un inhibiteur des monoamines oxydases.

9. Composé pour utilisation ou utilisation selon la revendication 8 **caractérisé en ce que** le composé est choisi parmi la moclobémide, la phénelzine, l'harmaline, la nialamide, la sélégiline, l'isocarboxazide, l'iproniazide, l'iproclozide, la toloxatone et la tranylcypromine.

10. Composé pour utilisation selon la revendication 1 ou utilisation selon la revendication 2 **caractérisé en ce que** le composé est choisi parmi la fluoxétine sous forme chlorhydrate, l'imipramine sous forme chlorhydrate, l'amitriptyline sous forme chlorhydrate, la désipramine sous forme chrorhydrate et la trimipramine sous forme maléate.

11. Composé pour utilisation selon l'une des revendications 1 et 3 à 10 pour son utilisation dans le traitement ou la prévention de la myopathie de Duchenne.

12. Utilisation selon l'une des revendications 2 à 10 **caractérisée en ce que** le médicament est destiné au traitement ou à la prévention de la myopathie de Duchenne.

## Patentansprüche

1. Verbindung, die eine Erhöhung des Spiegels von freiem Serotonin im Organismus bewirkt, für ihre Verwendung bei der Behandlung oder der Prävention von einer Muskeldegeneration der Skelettmuskulatur entsprechenden Pathologien, die aus den Muskelmyopathien genetischen Ursprungs ausgewählt sind.

2. Verwendung einer Verbindung, die eine Erhöhung des Spiegels von freiem Serotonin im Organismus bewirkt, für die Herstellung eines Medikaments, das für die Behandlung oder die Prävention von einer Muskeldegeneration der Skelettmuskulatur entsprechenden Pathologien, die aus den Muskelmyopathien genetischen Ursprungs ausgewählt sind, bestimmt ist.

3. Verbindung zur Verwendung nach Anspruch 1 oder Verwendung nach Anspruch 2, **dadurch gekennzeichnet, dass** die Verbindung ein selektiver Serotonin-Wiederaufnahme-Hemmer ist.

4. Verbindung zur Verwendung oder Verwendung nach Anspruch 3, **dadurch gekennzeichnet, dass** die Verbindung ausgewählt ist aus: Citalopram, Fluoxetin der Formel: 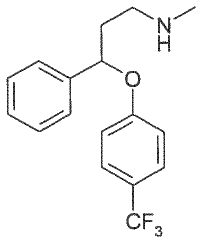 insbesondere in Chlorhydratform, Norfluoxetin, Fluvoxamin insbesondere in Maleatform, Escitalopram insbesondere in Oxalatform, Paroxetin, Sertralin, Alaproclat, Etoperidon, Escitalopram und Zimelidin.

5. Verbindung zur Verwendung nach Anspruch 1 oder Verwendung nach Anspruch 2, **dadurch gekennzeichnet, dass** die Verbindung eine tricyclische oder tetracyclische Verbindung ist, die die Wiederaufnahme verschiedener Neurotransmitter, darunter Serotonin, Noradrenalin und Dopamin, verhindert.

6. Verbindung zur Verwendung oder Verwendung nach Anspruch 5, **dadurch gekennzeichnet, dass** die Verbindung zu der Familie der Phenothiazine oder der Dibenzazepine gehört.

7. Verbindung zur Verwendung oder Verwendung nach Anspruch 5, **dadurch gekennzeichnet, dass** die Verbindung ausgewählt ist aus Amitriptylin, insbesondere in Chlorhydratform, der Formel: 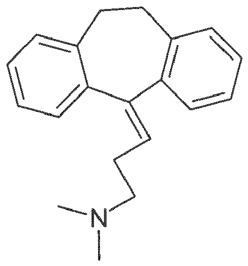 Clomipramin, Desipramin, insbesondere in Chlorhydratform, der Formel: 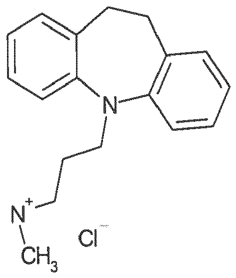 Dothiepin, Doxepin, Imipramin, insbesondere in Chlorhydratform, der Formel: 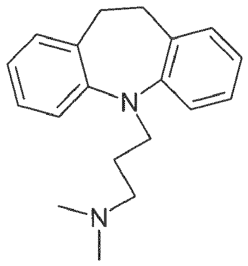 Lofepramin, Nortriptylin, Protriptylin, Trimipramin, insbesondere in Maleatform, der Formel: 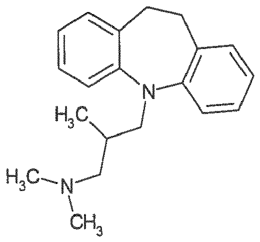 Iprindol, Opipramol, Maprotilin, Mianserin, Mirtazapin und Amoxapin.

8. Verbindung zur Verwendung nach Anspruch 1 oder Verwendung nach Anspruch 2, **dadurch gekennzeichnet, dass** die Verbindung ein Monoaminoxidase-Hemmer ist.

9. Verbindung zur Verwendung oder Verwendung nach Anspruch 8, **dadurch gekennzeichnet, dass** die Verbindung aus Moclobemid, Phenelzin, Harmalin, Nialamid, Selegilin, Isocarboxazid, Iproniazid, Iproclozid, Toloxaton und Tranylcypromin ausgewählt ist.

10. Verbindung zur Verwendung nach Anspruch 1 oder Verwendung nach Anspruch 2, **dadurch gekennzeichnet, dass** die Verbindung aus Fluoxetin in Chlorhydratform, Imipramin in Chlorhydratform, Amitriptylin in Chlorhydratform, Desipramin in Chlorhydratform und Trimipramin in Maleatform ausgewählt ist.

11. Verbindung zur Verwendung nach einem der Ansprüche 1 und 3 bis 10 für ihre Verwendung bei der Behandlung oder der Prävention der Muskeldystrophie vom Typ Duchenne.

12. Verwendung nach einem der Ansprüche 2 bis 10, **dadurch gekennzeichnet, dass** das Medikament für die Behandlung oder die Prävention der Muskeldystrophie vom Typ Duchenne bestimmt ist.

## Claims

1. A compound which induces an increase in the free serotonin level in the body, for use thereof in the treatment or prevention of pathologies corresponding to muscular degeneration of the skeletal muscles selected from among muscular myopathies of genetic origin.

2. Use of a compound which induces an increase in the free serotonin level in the body for preparing a drug for the treatment or prevention of pathologies corresponding to muscular degeneration of the skeletal muscles selected from among muscular myopathies of genetic origin.

3. A compound for use according to claim 1, or use according to claim 2, **characterized in that** the compound is a selective inhibitor of serotonin reuptake.

4. A compound for use according to claim 3 **characterized in that** the compound is selected from among: citalopram, fluoxetine of formula: 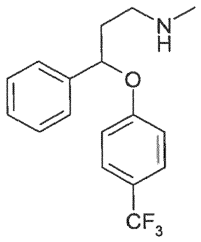 in particular in hydrochloride form, norfluoxetine, fluvoxamine in particular in maleate form, escitalopram in particular in oxalate form, paroxetine, sertraline, alaproclate, etoperidone, escitalopram and zimelidine.

5. A compound for use according to claim 1, or use according to claim 2, **characterized in that** the compound is a tricyclic or tetracyclic compound which prevents the reuptake of various neurotransmitters including serotonin, noradrenaline and dopamine.

6. A compound for use or use according to claim 5, **characterized in that** the compound belongs to the family of phenothiazines or dibenzazepines.

7. A compound for use or use according to claim 5, **characterized in that** the compound is selected from among amitriptyline, in particular in hydrochloride form, of formula: 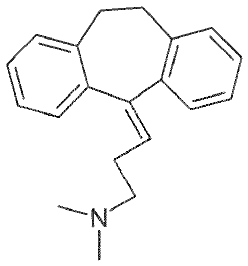 clomipramine, desipramine in particular in hydrochloride form, of formula: 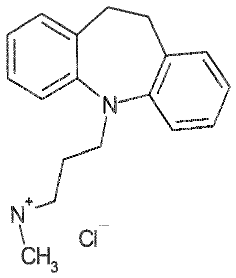 dothiepin, doxepin, imipramine, in particular in hydrochloride form, of formula: 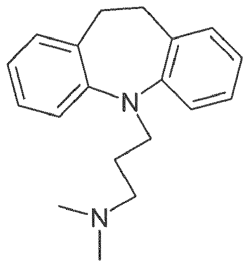 lofepramine, nortriptyline, protriptyline, trimipramine in particular in maleate form of formula: 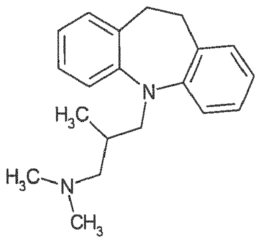 irprindole, opipramol, maprotiline, mianserin, mirtazapine and amoxapine.

8. A compound for use according to claim 1, or use according to claim 2, **characterized in that** the compound is an inhibitor of monoamine oxidases.

9. A compound for use or use according to claim 8, **characterized in that** the compound is selected from among moclobemide, phenelzine, harmaline, nialamide, selegiline, isocarboxazide, iproniazid, iproclozid, toloxatone and tranylcypromine.

10. A compound according to claim 1 or use according to claim 2, **characterized in that** the compound is selected from among fluoxetine in hydrochloride form, imipramine in hydrochloride form, amitriptyline in hydrochloride form, desipramine in hydrochloride form and trimipramine in maleate form.

11. A compound according to one of claims 1 and 3 to 10 for use thereof in the treatment or prevention of Duchenne muscular dystrophy.

12. The use according to one of claims 2 to 10, **characterized in that** the drug is for the treatment or prevention of Duchenne muscular dystrophy.
